# EUROPÄISCHE PATENTSCHRIFT

(11) **EP 0 245 840 B1**
(45) Veröffentlichungstag und Bekanntmachung des Hinweises auf die Patenterteilung: **01.12.1993**
(21) Anmeldenummer: 87106873.0
(22) Anmeldetag: 12.05.1987
(51) Int. Cl.: C08G 69/02, C08G 69/10, A61K 9/22, A61K 47/00

(54) **Biologisch abbaubare Polymere für Depotzubereitungen mit kontrollierter Wirkstoffabgabe**
Biodegradable polymers for retarding preparations with controlled release
Polymères biodégradables pour préparations retard à libération contrôlée

(30) Priorität: 15.05.1986 DE 3616320; 07.03.1987 DE 3707369
(43) Veröffentlichungstag der Anmeldung: 19.11.1987
(73) Patentinhaber: HOECHST AKTIENGESELLSCHAFT, 65926 Frankfurt am Main (DE)
(72) Erfinder: Bader, Hubert, Dr., D-6500 Mainz (DE); Rüppel, Diether, Dr., D-6230 Frankfurt am Main 80 (DE); Walch, Axel, Dr., D-6000 Frankfurt am Main (DE); Magerstädt, Michael, Dr., D-6237 Liederbach (DE)

(56) Entgegenhaltungen:
- WO-A-78/00011

## Beschreibung

Eine langandauernde kontrollierte Wirkstoffgabe ist wegen der zunehmenden Bedeutung chronischer Erkrankungen und langzeitorientierter Therapiekonzepte in Human- und Veterinärmedizin von großer Aktualität.

Arzneiabgabesysteme, in denen der Wirkstoff in einer nichtabbaubaren Polymermatrix dispergiert ist und dadurch Diffusion freigesetzt wird, sind in der Amerikanischen Patentschrift 4,069,307 beschrieben. Nach Erschöpfung des Wirkstoffreservoirs müssen solche Implantate jedoch operativ aus dem Organismus entfernt werden.

In biologisch abbaubaren Arzneistoffabgabesystemen, wie in dem Amerikanischen Patent 4,093,709 angegeben, wird der Wirkstoff in einem bioabbaubaren Polymeren dispergiert, welches beim Abbau den Wirkstoff freigibt. Typische, nach dem Stand der Technik meist untersuchte, biologisch abbaubare Polymere sind Homo- und Copolyester, insbesondere der Milch- und Glycolsäure, wie sie in den US-Patenten 3,773,919 bzw. 3,297,033 beschrieben sind. Nachteilig ist u.a. die geringe oder schlecht kontrollierbare Quellbarkeit der Polyester im physiologischen Milieu, welche die Permeation der im Implantat inkorporierten Wirkstoffe behindert und eine nach initialem "burst-effect" nur geringe Freisetzungsrate bewirkt. In neuerer Zeit sind in dem US Patent 4,304,767 Polyacetale und -ketale, bzw. Polyanhydride von H.G. Rosen et al., Biomaterials 4, 131 (1983), und Polyorthoester in dem US Patent 4,180,646 beschrieben worden, die als biologisch abbaubare Polymere für den Einsatz als Implantatmaterialien entwickelt wurden. Der Abbau dieser Polymere ist wegen des Fehlens weiterer funktioneller Gruppen, ähnlich den angeführten Polyestern, nur durch die hydrolytische Beständigkeit der Carbonylfunktion in der Polymer-Hauptkette bestimmt Solche Polymere verfügen außerdem nicht über eine für Implantationszeiträume von Monaten ausreichende Stabilität. Als weitere Polymerklasse sind in der Amerikanischen Patentschrift 3,371,069 Polyamide, insbesondere Polyaminosäuren, als bioresorbierbare Implantatmaterialien beschrieben worden. Die technische Herstellung von Polyaminosäuren erfordert jedoch den Einsatz teurer geschützter Aminosäuren, größere Mengen an hochgiftigem Phosgen, die Abspaltung der Schutzgruppen und die chemische Modifizierung der erhaltenen Polymere.

Ferner offenbart die Patentanmeldung WO 78/00 011 Polymere, die durch Polykondensation von bifunktionellen Carbonsäuren mit Polyolen hergestellt werden. Zur Polykondensation können auch Aminosäuren, wie Lysin, verwendet werden.

Überraschend wurde gefunden, daß Polyamide, in denen Aminosäuren über zwei Amin- oder Carboxylgruppen in das Polymerrückgrat eingebaut sind und die in α-Position zur Amidstruktur eine für Abbau und Wirkstoffabgabe verantwortliche funktionelle Gruppe tragen, sich hervorragend zum Einsatz als abbaubare Arzneistoffimplantate mit kontrollierter Wirkstoffabgabe eignen. Diese biologisch abbaubaren Polymere werden durch Polykondensation von physiologischen und pharmakologisch akzeptablen Diaminen mit ebensolchen Dicarbonsäuren erhalten. In vivo werden diese Polymere zu untoxischen, nicht-allergenen und nicht-immunogenen Verbindungen metabolisiert und ausgeschieden.

Die Erfindung betrifft somit:
1) Ein biologisch abbaubares Polyamid, in denen von Ia und/oder IIa abgeleitete monomere Verbindungen über zwei Amin- oder Carboxylgruppen in das Polymerrückgrat eingebaut sind und die in α-Position zur Amidstruktur eine für Abbau und Wirkstoffabgabe verantwortliche funktionelle Gruppe tragen, mit
   I) wiederkehrenden Einheiten der Diaminoverbindung aus der Gruppe
      - der monomeren Verbindung der allgemeinen Formel Ia, 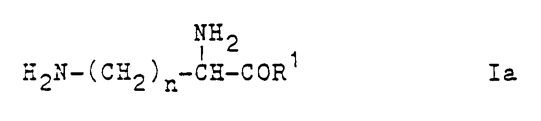
         - in der R¹: eine physiologisch unbedenkliche, hydrolysierbare Alkoxygruppe mit bis zu 18 C-Atomen, die gegebenenfalls mit physiologisch unbedenklichen, Seitengruppen substituiert sein kann, oder eine physiologisch unbedenkliche, hydrolysierbare Alkylamino- oder Aralkylaminogruppe bedeutet und
         - n: 3 oder 4 ist,
         und/oder
      - der monomeren Verbindung, die durch Veresterung von Aminoethanol mit den Dicarbonsäuren des Citratcyklus entsteht, und/oder
      - der monomeren Verbindung der allgemeinen Formel Ib, 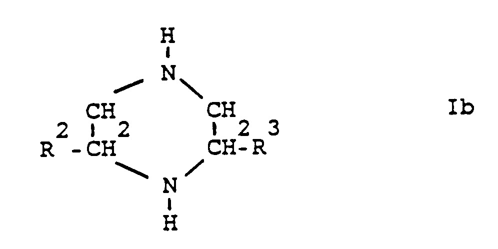
         - in der R² und R³: unabhängig voneinander Wasserstoff oder Methyl bedeuten
         und
   II) wiederkehrenden Einheiten der Dicarbonsäure-Verbindungen aus der Gruppe
      - der monomeren Verbindung der allgemeinen Formel IIa, 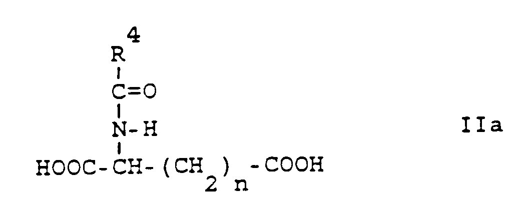
         - in der R⁴: eine Alkylgruppe mit 1 bis 3 C-Atomen und
         - n: 1 oder 2
         bedeutet und/oder
      - der monomeren geradkettigen, gesättigten oder 1-fach ungesättigten Dicarbonsäuren mit 2-10 Kohlenstoffatomen
         und/oder
      - der monomeren Verbindung, die durch Veresterung der Dicarbosäuren des Citratcyklus mit Diolen der allgemeinen Formel IIb entsteht, 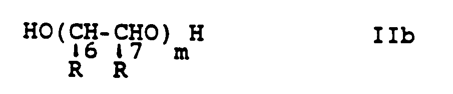
         - in der R⁶ und R⁷: unabhängig voneinander Wasserstoff oder eine Methylgruppe bedeutet und
         - m: eine Zahl im Bereich von 1 bis 100 ist
         oder durch Amidierung mit Diaminen der genannten allgemeinen Formel Ib.
2) Das Verfahren zur Herstellung des obengenannten Polyamids, das dadurch gekennzeichnet ist, daß eine oder mehrere der unter 1) genannten Diamino- und Dicarbonsäure-Verbindungen polykondensiert werden.
3) Die Verwendung des obengenannten Polyamids zum Einkapseln von biologisch aktiven Substanzen.
4) Die Verwendung des obengenannten Polyamids als abbaubare Wirkstoffdepotzubereitung mit kontrollierter Wirkstoffabgabe.

Im folgenden wird die Erfindung detailliert beschrieben bzw. in den Ansprüchen definiert.

Als Diaminoverbindung der Formel Ia werden Ester des Ornithin und Lysin mit physiologisch unbedenklichen, hydrolysierbaren Alkoxygruppen eingesetzt, die bis zu 18 C-Atome besitzen. Ester mit höheren Alkoxygruppen können zwar auch noch verwendet werden, mit zunehmender Kettenlänge wird jedoch die Polymerisation erschwert. Diese Alkoxygruppen können gegebenenfalls mit hydrolysierbaren, physiologisch unbedenklichen Seitengruppen substituiert sein. Geeignet sind beispielsweise folgende Alkoxygruppen:
- n- bzw. iso- (C₁-C₁₈) Alkyloxy, bevorzugt Methoxy, Ethoxy, Butyloxy, Octadecyloxy, Isopropyloxy;
- Methoxy(C₂-C₄)alkyloxy, bevorzugt Methoxypolyethylenoxy, Methoxypolypropylenoxy;
- Hydroxy(C₂-C₄)alkyloxy, bevorzugt 2-Hydroxy-1-propyloxy, 2-Hydroxy-3-butyloxy;
- Trichlorisobutyloxy;
- (C₂-C₄)Alkoxycarbonylalkylenoxy, bevorzugt Ethoxycarbonylmethylenoxy, Butoxycarbonylmethylenoxy.

Besonders bevorzugt werden die Methyl-, Ethyl- und n-Butyloxygruppen verwendet.

Anstelle der Alkoxygruppen sind auch hydrolysierbare, physiologisch unbedenkliche Alkylamino- oder Aralkylaminogruppen geeignet, wie beispielsweise folgende Gruppen:
- Hydroxy(C₂-C₆)alkylamino, bevorzugt 2-Hydroxyethylamino, Tris(hydroxymethylen)methylamino, Glycosylamino;
- (C₂-C₄)Alkanoyloxyethylamino, bevorzugt 2-Acetoxyethylamino, 2-Butanoyloxyethylamino;
- Mercapto(C₂-C₄)alkylamino, bevorzugt 2-Mercaptoethylamino; die Methylester der natürlichen α-Aminosäuren, bevorzugt Phenylalaninmethylester und Leucinmethylester.
Die Gruppe R¹ kann wie oben angedeutet sehr variabel sein, inbesondere da sie in dem eigentlichen erfindungsgemäßen Polymer nur als Seitengruppe auftritt. Sie kann beispielsweise auch eine Hydroxylgruppe sein, wenn Copolyamide oder Polymermischungen eingesetzt werden.

Durch Veresterung von Aminoethanol mit den Dicarbonsäuren des Citratcyklus, insbesondere Bernsteinsäure und Fumarsäure, erhält man ebenfalls Diamine, die erfindungsgemäß eingesetzt werden. Weiterhin können pharmakologisch akzeptable Piperazine der Formel Ib Anwendung finden.

Erfindungsgemäß Dicarbonsäuren sind N-acylierte Glutaminsäure und Asparaginsäure der Formel IIa, deren Aminogruppe durch eine (C₂-C₄)Acylgruppe geschützt ist. Bevorzugte Acylgruppen sind Acetyl und Butyryl, insbesondere Acetyl. Die Acylgruppen können auch längerkettig sein, allerdings wird es mit zunehmender Kettenlänge aufwendiger, sie in die Dicarbonsäure einzuführen. Außerdem wird die Reaktivität der α-ständigen Carbonylgruppe durch längerkettige Acylgruppe herabgesetzt.

Weiterhin können auch geradkettige, gesättigte oder 1-fach ungesättigte Dicarbonsäuren mit 2-10, bevorzugt 4-8, besonders bevorzugt 4-6 Kohlenstoffatomen zur Polykondensation eingesetzt werden. Hierbei sind wieder die Glutarsäure und die Fumarsäure ganz besonders bevorzugt. Weiterhin ist es möglich, durch Veresterung dieser Dicarbonsäuren mit pharmakologisch verträglichen Diolen der Formel IIb, bzw. Durch Amidierung mit Diaminen der Formel Ib Dicarbonsäureverbindungen herzustellen, die ebenfalls erfindungsgemäß Anwendung finden können. Geeignete Diole sind beispielsweise Propandiol und Ethylenglykol sowie deren Polymere mit bis zu 100 wiederkehrenden Einheiten. Vorzugsweise wird 2,3-Butandiol verwendet. In diesem Zusammenhang geeignete Diamine sind Piperazin und seine physiologisch akzeptablen Methylhomologen. Bevorzugt kommt jedoch Piperazin zur Anwendung.

Die erfindungsgemäßen biologisch abbaubaren Polyamide werden nach an sich bekannten Methoden kondensiert (P.W. Morgan "Condensation Polymers: by Interfacial and Solution Methods" Interscience Publ., New York, 1965). Durch Grenzflächenpolykondensation der freien Diamine mit Dicarbonsäurechloriden bzw. durch Lösungspolykondensation der Diamine oder ihrer Bis-silylderivate mit Chloriden oder Aktivestern der Dicarbonsäuren in einem aprotisch dipolaren Lösungsmittel sind diese Produkte leicht herzustellen.

Dazu wird die Diaminokomponente in Wasser gelöst, das überschüssiges Diamin, organische oder anorganische Basen wie z.B. Trialkylamine oder Alkalihydroxide oder Alkalicarbonate als Säurefänger enthält. Die Dicarbonsäurekomponente, vorzugsweise das Dicarbonsäuredichlorid wird in mit Wasser nicht mischbaren organischen Lösungsmitteln wie z.B. aliphatischen, cycloaliphatischen und aromatischen Kohlenwasserstoffen oder aliphatischen und aromatischen Halogenkohlenwasserstoffen gelöst. Diese Lösung wird unter starkem Rühren zur Diaminlösung zugegeben und das entstandene Polymer durch Filtration oder Zentrifugation isoliert. Das Polyamid wird mit Wasser und Ethanol oder Aceton gewaschen und im Vakuum bei erhöhter Temperatur getrocknet.

Das derart hergestellte Polyamid besteht zu 50% aus wiederkehrenden Einheiten der Diaminoverbindungen und zu 50% aus wiederkehrenden Einheiten der Dicarbonsäureverbindungen. Es können Homopolymere aufgebaut werden, d.h. je eines der angegebenen Diaminoverbindungen reagiert mit einer der angegebenen Dicarbonsäureverbindungen, aber auch Copolymere, in denen zwei oder mehrere Verbindungen aus der Gruppe der Diaminoverbindungen sowie zwei oder mehrere Verbindungen aus der Gruppe der Dicarbonsäureverbindungen enthalten sind.

Bei Verwendung der Aminoverbindung der allgemeinen Formel Ia und der Fumarsäure oder dem Gemisch Fumarsäure/Glutarsäure als Dicarbonsäure zur Polykondensation sind die entsprechenden Produkte über die Esterfunktion des Lysin-oder Ornithinrestes polymeranalogen Umsetzungen mit Aminen, insbesondere mit Aminoethanol, oder aminofunktionellen Pharmaka bzw. über die Doppelbindungen des Fumarsäureesters einer radikalischen Quervernetzung zugänglich. Auf diese Weise können physikalische Eigenschaften, z.B. Löslichkeit und Hydrophilie, und physiologische Eigenschaften, wie Stabilität gegenüber hydrolytischem Abbau, Verträglichkeit oder pharmakologische Aktivität, nachträglich am Polymeren noch modifiziert werden.

Die zur Amidstruktur α-ständige Carbonyl- oder Aminoacylfunktion beeinflußt durch induktive und sterische Effekte den Abbau des Polymeren und die Wirkstoffabgabe. Beispielsweise wird nach bekannten enzymatischen oder hydrolytischen Esterspaltungen im Körper durch die entstehende Carboxylgruppe die Hydrophilie des Polymeren und damit seine Quellbarkeit (Wasserresorption und Löslichkeit im physiologischen Milieu erhöht. Dadurch wird der bei den beschriebenen Implantat-Materialien nach initialem "burst-effect" auftretende unerwünschte, starke Abfall der Freisetzungsrate kompensiert.

Aus den erfindungsgemäßen Polyamiden können nach bekannten Methoden implantierbare Partikel, insbesondere Mikrokapseln und Mikrosphären sowie, durch Kompaktierung makroskopische Formkörper beliebiger Geometrie, insbesondere Tabletten und Stäbchen hergestellt werden.

Das Polyamid kann beispielsweise mit dem Wirkstoff in einem geeigneten polaren aprotischen Lösungsmittel, beispielsweise Dimethylsulfoxid oder Dimethylacetamid, gelöst werden. Die Lösung wird unter Zugabe eines Emulgators in eine Ölphase bei einer Temperatur emulgiert, bei der die Polymerlösung verflüssigt ist. Nach einigen Minuten werden die einzelnen Lösungsmittel/Polymer Tröpfchen durch Abkühlen der Emulsion zum Erstarren gebracht. Durch Waschen mit einem geeigneten Lösungsmittel, in dem sich das zur Lösung des Polyamids eingesetzte Lösungsmittel sowie die Ölphase lösen, die Polymertröpfchen jedoch nicht, werden die Polymerkugeln ausgehärtet. Dabei verkleinert sich ihr Volumen, die Form ändert sich nicht.

Die erfindungsgemäßen Polyamide können auch als Gemische und in Abmischungen mit anderen bioabbaubaren Polymeren oder physiologisch unbedenklichen Hilfsstoffen (z.B. Polymerweichmacher) eingesetzt werden.

Abbauversuche in vitro mit den erfindungsgemäßen Polyamiden haben gezeigt, daß die Abbaurate über die funktionellen Seitengruppen kontrolliert gesteuert werden kann.

In den folgenden Beispielen wird die Erfindung im einzelnen beschrieben. Prozentangaben beziehen sich auf das Gewicht, sofern nicht anders angegeben.

### Beispiel 1

### Herstellung von Poly(L-Lysinethylesterfumaramid) (LEF)

0,76 g Fumarsäurechlorid in 100 ml ethanolfreiem Chloroform wird unter schnellem Rühren zu einer Lösung von 2,47 g L-Lysinethylester-dihydrochlorid und 2,12 g Natriumcarbonat in 100 ml eiskaltem Wasser gegeben. Nach zehnminütigem Rühren bei Raumtemperatur werden 100 ml 1N Salzsäure zugegeben und noch eine Minute weitergerührt. Das entstandene Polymer wird über eine Glasfilterfritte abgesaugt und erst mit heißen Wasser, dann mit kaltem Wasser und anschließend mit Aceton gewaschen. Nach Trocknung im Vakuum über Phosphorpentoxid erhält man 0,8 - 0,95 g (63 - 75% d.Th.) weißes Poly-L-Lysinethylesterfumaramid. (M_{w} 23.000, Wasserresorption 9,5 Gew.%, T_{G} 75°C).

### Beispiel 2

### Herstellung von Poly(L-Lysinmethylesterfumaramid) (LMF)

Analog Beispiel 1 wird 0,76 g Fumarsäurechlorid mit 2,34 g L-Lysinmethylesterdihydrochlorid polykondensiert. Man erhält 0,8 g weißes Poly-L-Lysinmethylesterfumaramid (LMF).

### Beispiel 3

### Herstellung von Poly(L-Lysinbutylesterfumaramid) (LBF)

1,52 g Fumarsäurechlorid in 200 ml über Phosphorpentoxid destilliertem Methylenchlorid werden unter Rühren mit einem Ultraturax zu einer Lösung von 5,5 g L-Lysinbutylesterdihydrochlorid und 4,24 g Natriumcarbonat in 200 ml eiskaltem Wasser gegossen. Nach zweiminütigem Rühren unter Eiskühlung werden 100 ml 1 N Salzsäure zugegeben und noch eine Minute gerührt. Das Methylenchlorid wird durch Einleiten von Dampf aus dem Reaktionsgemisch ausgetrieben und das Polykondensat anschließend über eine Fritte abgesaugt. Nach Waschen mit heißem Wasser, kaltem Wasser und Ethanol wird das Polymer im Vakuum bei 60°C getrocknet. Man erhält 1,8 g (64 % d.Th.) weißes, faseriges Poly (L-Lysinbutylesterfumaramid).

### Beispiel 4

### Herstellung von Copoly(L-Lysinmethylester-piperazinfumaramid)

Analog Beispiel 2 wird 0,76 g Fumarsäurechlorid in 100 ml Methylenchlorid mit 1,2 g L-Lysinmethylesterdihydrochlorid und 0,93 g Piperazin in 100 ml Wasser, das 2,12 g Natriumcarbonat enthält, zur Polykondensation gebracht, Man erhält 0.7 g (69 % d.Th.) des Copolyamids, das sich in konz. Schwefelsäure und Ameisensäure löst.

### Beispiel 5

### Herstellung von Copoly(L-Lysinethyl-butylesterfumaramid)

Analog Beispiel 2 werden 1,52 g Fumarsäurechlorid in 200 ml Methylenchlorid mit 2,47 g Lysinethylesterdihydrochlorid und 2,75 g Lysinbutylesterdihydrochlorid in 200 ml Wasser, das 5,5 g Kaliumhydroxid enthält, polykondensiert. Es werden 2,5 g (71 % d.Th.) des weißen, faserartigen Copolykondensats erhalten.

### Beispiel 6

### (LMEF 75/25)

Analog Beispiel 4 werden 1,52 g Fumarsäurechlorid mit 3,45 Lysinmethylesterdihydrochlorid und 1,30 g Lysinethylesterdihydrochlorid polykondensiert. Es werden 2,0 g (70 % d.Th. eines weißen, faserartigen Copolykondensats erhalten.

### Beispiel 7

### (LMFG 50/50)

### Herstellung von Poly (L-Lysinmethylesterfumaramid/L-Lysinmethylesterglutaramid)

### Copolymer 50 : 50 (LMFG 50 : 50)

0,7 g Glutarsäuredichlorid und 0,84 g Fumarsäuredichlorid werden in 170 ml CH₂Cl₂ gelöst. Diese Lösung wird bei Raumtemperatur unter kräftigem Rühren zu einer Lösung von 2,91 g L-Lysinmethylester und 3,0 g Na₂CO₃ in 120 ml H₂O gegeben. Die schlagartig eintretende Polykondensation wird nach 15 Minuten durch Zugabe von 120 ml 1 N wäßriger HCl abgebrochen. Durch Einleiten von Wasserdampf wird dann das Methylenchlorid ausgetrieben. Das heiße wäßrige Gemisch wird filtriert und das feste Produkt mehrmals mit Wasser gewaschen und mit siedendem Ethanol trituriert.
Ausbeute: 1,3 g (60 % d.Th.) nach Vakuumtrocknung (20 h).

### Beispiel 8

### (LMFG 60/40)

### Herstellung von Poly (L-Lysinmethylesterfumaramid/L-Lysinmethylesterglutaramid)

### Copolymer 60 : 40 (LMFG 60 : 40)

0,56 g Glutarsäuredichlorid und 0,77 g Fumarsäuredichlorid werden in 170 ml CH₂Cl₂ gelöst und wie in Beispiel 7 beschrieben, mit eine Lösung von 2.91 g L-Lysinmethylester und 3 g Na₂CO₃ in 120 ml H₂O polykondensiert.
Ausbeute: 1,3 g (60 % d.Th.).

### Beispiel 9

### Herstellung monolithischer Mikrokugeln aus LEF

460 mg Octadecanol werden in 100 ml dickflüssigem Paraffin (Fa. Riedel de Haen) durch Ultraschallbehandlung gelöst, auf 50°C erwärmt und gerührt. 70 mg LEF, das gemäß Beispiel 1 hergestellt wurde, und 30 mg LHRH-analoges Peptidhormon werden in Dimethylsulfoxid durch Ultraschall gelöst. Die Lösung wird tropfenweise in die gerührte Paraffinlösung gegeben und 10 min emulgiert.

Die Emulsion wird bei 40°C in 300 ml n-Butanol eingerührt, wobei sich die Paraffinmatrix auflöst und die Polymerkugeln ausfallen. Nach ca. 6 h wird abdekantiert, die Polymerkugeln werden in 100 ml Butanol aufgenommen, 16 h ausgehärtet und danach abzentrifugiert.
Man erhält Polymerkugeln mit einer Größenverteilung zwischen 20 und 100 µm.

### Beispiel 10

### Herstellung monolithischer Mikrokugeln aus LMFG 60 : 40 (aus Beispiel 8)

90 mg LMFG 60 : 40 aus Beispiel 8 werden in 1 ml Dimethylsulfoxid in kleinen Portionen gelöst. Die Lösung wird aus einer Spritze mit feiner Kanüle (Kanülendurchmesser außen 0,4 - 1,2 mm: abhängig von gewünschter Kugelgröße) in ca. 50 ml flüssigen Stickstoff getropft. Die so entstehenden gefrorenen Kugeln werden durch Dekantieren vom Stickstoff getrennt und in ca. 500 ml Wasser gegeben. Nach 2 h ist das DMSO aus den Kugeln diffundiert und die Kugeln sind ausgehärtet. Sie werden 20 h gefriergetrocknet.

### Beispiel 11

### Herstellung von Mikrospheres mit Wirkstoff

56 mg LMF aus Beispiel 2 und 14 mg ®Pluronic F 68 (Hersteller Fluka AG, Neu-Ulm) werden in 1 ml Dimethylsulfoxid bei 50°C gelöst. Danach werden 30 mg Buserelin (Hersteller Behringwerke AG, Marburg) durch kurzes Beschallen mit Ultraschall gelöst. Die Polymer- und Wirkstofflösung wird mit einer Kanüle (Einwegspritze, Kanülendurchmesser außen 0,6 mm) in eine Vorlage von flüssigem Stickstoff (100 ml) eingetropft.
Die entstandenen Mikrospheres werden in 200 ml Wasser überführt und 2 Stunden von restlichem Lösungsmittel extrahiert. Überschüssiges Wasser wird abdekantiert und die Mikrospheres lyophilisiert (Durchmesser nach Lyophilisation 1-2 mm).

### Beispiel 12

### Herstellung von Mikrospheres

70 mg LMF aus Beispiel 2 und 30 mg Maisstärke SF Typ Snowflake 05063 (Hersteller Maizena Industrieprodukte GmbH, Hamburg) werden in 1 ml Dimethylsulfoxid bei 50°C gelöst. Die Polymerlösung wird mit einer Kanüle (Einwegspritze, Kanülendurchmesser außen 0,6 mm) in eine Vorlage von flüssigem Stickstoff (100 ml) eingetropft.
Die entstandenen Mikrospheres wurden in 200 ml Wasser überführt und 2 Stunden von restlichem Lösungsmittel extrahiert und die Mikrospheres lyophilisiert (Durchmesser nach Lyophilisation 1-2 mm).

### Beispiel 13

### Abbauversuche

5 Proben von jeweils 100 mg Poly-lysinethylfumaramid, das gemäß Beispiel 1 hergestellt wurde, werden jeweils in 5 Semi-Mikro-Dialysierschläuche aus regenerierter Cellulose (Spectra/ Por No. 132600, Spectrum Medical Ind., Inc.L.A., U.S.A) eingebracht. Die Schlauchsegmente (Länge 80 mm, Flachbreite 2,5 mm) werden mit einer Drahtschlinge verschlossen und unter Rühren in einer Phosphat-Pufferlösung aus 0,00205 mol Na₂HPO₄ und 0,0045 mol NaH₂PO₄ (pH 7,4) bei 37°C und einem Sauerstoff-Partialdruck von 50 mm Hg inkubiert.

Der Phosphatpuffer wird mit 0,108 mol NaCl und 0,030 mol NaHCO₃ versetzt und durch 0,0078 mol NaN₃ gegen den Befall von Mikroorganismen stabilisiert. Der Puffer wird mit einem Durchsatz von 50 ml/d ausgetauscht.

Über einen Zeitraum von 120 Tagen wird nach jeweils 30 Tagen eine Probe genommen, mit destilliertem Wasser gewaschen und das Abbauverhalten,wie in der nachstehenden Tabelle aufgeführt, anhand des verbleibenden Polymeren wie folgt charakterisiert:
a) Trockengewicht nach 50 h Lagerung über P₂O₅ im Vakuum
b) Wasserresorption nach 74 h Lagerung bei 92 % relativer Luftfeuchte
c) Molekulargewicht (ca M_{w}) durch Gelpermeationschromatographie mit Hilfe eines mit N,N'-Methylenbisacrylamid kovalent vernetzten Allyldextrans (^{(R)}Sephacryl S-200, Pharmacia, Uppsala) in Dimethylsulfoxid

| Tage | 30 | 60 | 90 | 120 |
|---|---|---|---|---|
| Trockengewicht (mg) | 85 | 79 | 68 | 55 |
| Wasserresorption (Gew.-%) | 17 | 25 | 32 | 38 |
| Mol.gewicht (Dalton) | 25000 | 22000 | 20000 | 19000 |

### Beispiel 14

Es wird die Wasserresorption (Gew.-%) verschiedener Homo- und Copolyamide nach 74 h Lagerung bei 92 % relativer Luftfeuchte bestimmt sowie die Dauer der Hydrolyse der Alkylester-Seitengruppe (h) bis zur vollständigen Solubilisierung von je 100 mg Polymerpulver in 10 ml wäßriger NaOH (pH 13).

| Polyamid | nach Beispiel | Wasserresorption | Solubilisierungsdauer [h] |
|---|---|---|---|
| Polylysinmethylesterfumaramid | 2 | 13,8 | 3 |
| Polylysinethylesterfumaramid | 1 | 9,2 | 48 |
| Polylysinbutylesterfumaramid | 3 | 5,7 | 864 |
| Polylysinmethyl/ethylesterfumaramid: Comonomer-Mol-Anteil | | 10,1 | 30 |
| 75 : 25 molar | 6 | | |
| Comonomer-Mol-Anteil | | 9,7 | 55 |
| 25 : 75 molar | 6 | | |
| Polylysinethyl/butylesterfumaramid: Comonomer-Mol-Anteil | | 8,2 | 528 |
| 75 : 25 molar | 5 | | |
| Comonomer-Mol-Anteil | | 7,0 | 696 |
| 50 : 50 molar | 5 | | |

### Beispiel 15

### Polymerabbau

Es wird die Wasserresorption (in Gew.-%) nach 74 h Lagerung bei 92 % relativer Luftfeuchte bestimmt sowie die Dauer der Hydrolyse der Seitengruppen (in Stunden) bis zur vollständigen Solubilisierung von je 100 mg Polymerpulver in 100 ml wäßriger NaOH (pH 13).
Der Abbau in Puffer bei physiologischem pH wird wie folgt durchgeführt.
Jeweils 500 mg Polymer werden in je 30 ml einer Phosphat-Pufferlösung aus 0,00205 mol Na₂HPO₄ und 0,0045 mol NaH₂PO₄ (pH 7,4) inkubiert und in verschlossenen Glasflaschen (50 ml) bei 37°C gerührt.

Der Phosphatpuffer wird mit 0,0078 mol NaN₃ gegen mikrobiellen Befall stabilisiert und nach jeweils 7 Tagen in seinem pH korrigiert.

Nach einem Zeitraum von 150 Tagen werden die Gewichtsabnahmen der Polymerproben gemessen: die Pufferlösung mit inkubiertem Polymer wird über eine tarierte Glasfritte filtriert, der Rückstand 24 h im Vakuum über Phosphorpentaoxid getrocknet und die Gewichtsabnahme bestimmt.

| Polymer | nach Beispiel | Wasserresorption % | Solubilisierung h | Gewichtsabnahme % |
|---|---|---|---|---|
| LMF | 2 | 14 | 3 | 21 |
| LMFG 60/40 | 8 | 18 | 1 | 38 |
| LEF | 1 | 9 | 48 | 6 |
| LMEF 75/25 | 6 | 10 | 30 | 9 |
| LMF/Pluronic F 68 | 11 | 16 | 0,5 | 55 |
| LMF/Stärke SF | 12 | 18 | 1 | 43 |

### Beispiel 16

### Release von Buserelin aus LMF/Pluronic F 68/Buserelin-Microspheres aus Beispiel 11

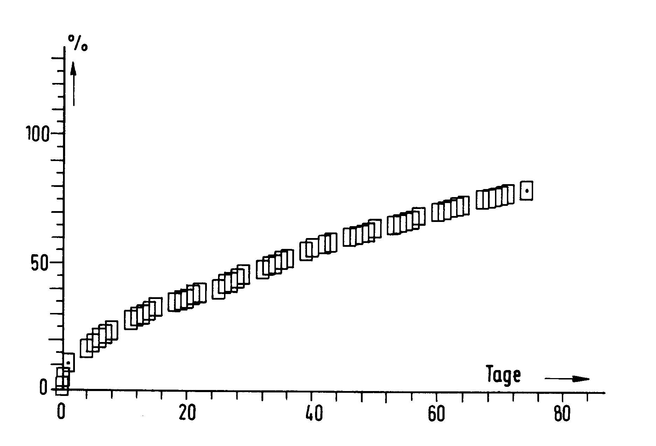

Die untersuchten Mikrokugeln haben die Zusammensetzung: 12 Gew.-% Buserelin, 25 Gew.-% Pluronic F 68, 63 Gew.-% LMF
Die Wirkstoffabgabe in eine Pufferlösung wurde UV-spektroskopisch gemessen. (Puffer: 2,91 g Na₂HPO₄; 0,540g NaH₂PO₄; 0,4 g NaN₃, 6,328 g NaCl; 2,52 g NaHCO₃ auf 1 l Wasser). In der Figur ist der gesamte, abgegebene Anteil von Buserelin in % als Funktion der Zeit (in Tagen) aufgetragen.

## Patentansprüche (Patentansprüche für folgende(n) Vertragsstaat(en): BE, CH, DE, FR, GB, GR, IT, LI, LU, NL, SE)

1. Biologisch abbaubares Polyamid, in denen von Ia und/oder IIa abgeleitete monomere Verbindungen über zwei Amin- oder Carboxylgruppen in das Polymerrückgrat eingebaut sind und die in α-Position zur Amidstruktur eine für Abbau und Wirkstoffabgabe verantwortliche funktionelle Gruppe tragen, das im wesentlichen je 50 Mol % der wiederkehrenden Einheiten I und II enthält, wobei
I) wiederkehrenden Einheiten abgeleitet von der Diaminoverbindung aus der Gruppe
- der monomeren Verbindungen der allgemeinen Formel Ia, 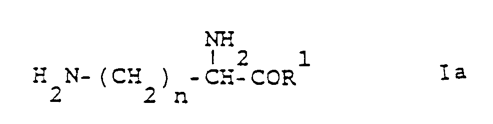
in der R¹ eine physiologisch unbedenkliche, hydrolisierbare Alkoxygruppe mit bis zu 18 C-Atomen, die gegebenenfalls mit physiologisch unbedenklichen Seitengruppen substituiert sein kann, oder
eine physiologisch unbedenkliche, hydrolisierbare Alkylamino- oder Aralkylaminogruppe bedeutet und
n 3 oder 4 ist,
und/oder
- der monomeren Verbindung, die durch Veresterung von Aminoethanol mit den Dicarbonsäuren des Citratcyklus entsteht, und/oder
- der monomeren Verbindung der allgemeinen Formal Ib 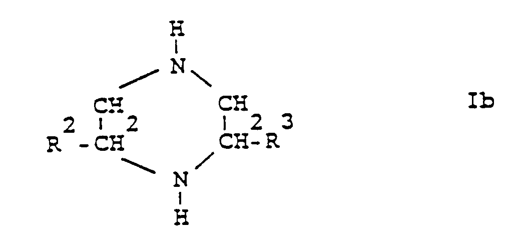
in der R² und R³ unabhängig voneinander Wasserstoff oder Methyl bedeuten und
II) wiederkehrenden Einheiten abgeleitet von der Dicarbonsäure-Verbindung aus der Gruppe
- der monomeren Verbindung der allgemeinen Formel IIa, 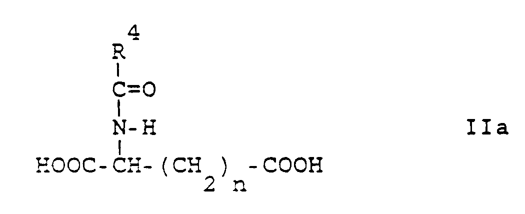
in der R⁴ eine Alkylgruppe mit 1 bis 3 C-Atomen
und
n 1 oder 2
bedeutet und/oder
- der monomeren geradkettigen, gesättigten oder 1-fach ungesättigten Dicarbonsäuren mit 2-10 Kohlenstoffatomen
und/oder
- der monomeren Verbindung, die durch Veresterung der Dicarbonsäuren des Citratcyklus mit Diolen der allgemeinen Formel IIb entsteht, 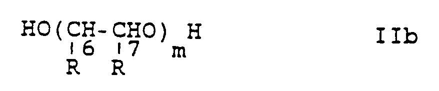
in der R⁶ und R⁷ unabhängig voneinander Wasserstoff oder eine Methylgruppe bedeutet
und
m eine Zahl im Bereich von 1 bis 100 ist
oder durch Amidierung mit Diaminen der genannten allgemeinen Formel Ib.

2. Polyamid nach Anspruch 1, dadurch gekennzeichnet, daß die Seitengruppe R¹ n- bzw. iso-(C₁-C₁₈)Alkyloxy, Methoxy(C₂-C₄)alkyloxy, Hydroxy(C₂-C₄)alkyloxy, (C₂-C₄) Alkoxycarbonylalkylenoxy, Trichlorisobutyloxy, Hydroxy(C₂-C₆)alkylamino, (C₂-C₄)Alkanoyloxyethylamino, Mercapto(C₂-C₄)alkylamino oder Methylester der natürlichen α-Aminosäuren bedeutet.

3. Polyamid nach Anspruch 1, dadurch gekennzeichnet, daß R¹ eine Methyl-, Ethyl- oder n-Butyloxygruppe bedeutet.

4. Polyamid nach Anspruch 1, dadurch gekennzeichnet, daß als Dicarbonsäuren geradkettige, gesättigte oder 1-fach ungesättigte Dicarbonsäuren mit 4-8 Kohlenstoffatomen eingesetzt werden.

5. Polyamid nach Anspruch 1, dadurch gekennzeichnet, daß als Dicarbonsäuren Fumarsäure und Glutarsäure eingesetzt werden.

6. Polyamid nach Anspruch 1, dadurch gekennzeichnet, daß die allgemeine Formel IIb 2,3-Butandiol bedeutet.

7. Verfahren zur Herstellung des Polyamids nach Anspruch 1, dadurch gekennzeichnet, daß je eine oder mehrere Verbindungen aus der Gruppe der Diamino- und Dicarbonsäure-Verbindungen polykondensiert werden.

8. Verwendung des Polyamids nach Anspruch 1 zum Einkapseln von biologisch aktiven Substanzen.

9. Verwendung des Polyamids nach Anspruch 1 zur Herstellung von biologisch abbaubaren Wirkstoffdepotzubereitungen mit kontrollierter Wirkstoffabgabe.

10. Verwendung des Polyamids nach Anspruch 1 in Abmischungen mit anderen bioabbaubaren Polyamiden oder physiologisch unbedenklichen Hilfsstoffen, zum Einkapseln von biologisch aktiven Substanzen.

11. Verwendung des Polyamids nach Anspruch 1 in Abmischungen mit anderen bioabbaubaren Polyamiden oder physiologisch unbedenklichen Hilfsstoffen, zur Herstellung von biologisch abbaubaren Wirkstoffdepotzubereitungen mit kontrollierter Wirkstoffabgabe.

## Patentansprüche (Patentansprüche für folgende(n) Vertragsstaat(en): AT)

1. Verfahren zur Herstellung eines biologisch abbaubaren Polyamids, in dem von Ia und/oder IIa abgeleitete monomere Verbindungen über zwei Amin- oder Carboxylgruppen in das Polymerrückgrat eingebaut sind und die in α-Position zur Amidstruktur eine für Abbau und Wirkstoffabgabe verantwortliche funktionelle Gruppe tragen, mit
I) wiederkehrenden Einheiten Einheiten der Diaminoverbindung aus der Gruppe
- der monomeren Verbindungen der allgemeinen Formel Ia, 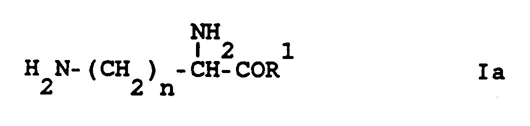
in der R¹ eine physiologisch unbedenkliche, hydrolysierbare Alkoxygruppe mit bis zu 18 C-Atomen, die gegebenenfalls mit physiologisch unbedenklichen, Seitengruppen substituiert sein kann, oder
eine physiologisch unbedenkliche, hydrolysierbare Alkylamino- oder Aralkylaminogruppe
und
n 3 oder 4 ist,
und/oder
- der monomeren Verbindung, die durch Veresterung von Aminoethanol mit den Dicarbonsäuren des Citratcyklus entsteht, und/oder
- der monomeren Verbindung der allgemeinen Formel Ib, 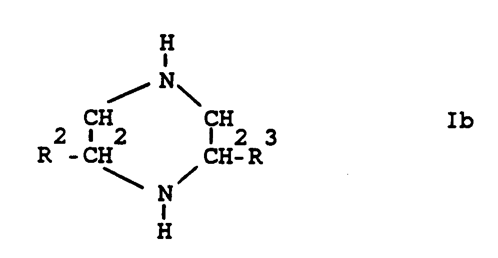
in der R² und R³ unabhängig voneinander Wasserstoff oder Methyl bedeuten
und
II) wiederkehrenden Einheiten der Dicarbonsäure-Verbindungen aus der Gruppe
- der monomeren Verbindung der allgemeinen Formel IIa, 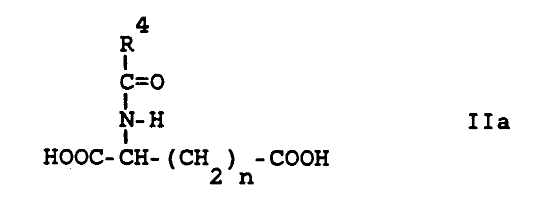
in der R⁴ eine Alkylgruppe mit 1 bit 3 C-Atomen
und
n 1 oder 2
bedeutet und/oder
- der monomeren geradkettigen, gesättigten oder 1-fach ungesättigten Dicarbonsäuren mit 2-10 Kohlenstoffatomen
und/oder
- der monomeren Verbindung, die durch Veresterung der Dicarbonsäuren des Citratcyklus mit Diolen der allgemeinen Formel IIb entsteht, 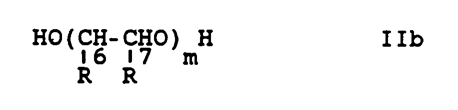
in der R⁶ und R⁷ unabhängig voneinander Wasserstoff oder eine Methylgruppe bedeutet
und
m eine Zahl im Bereich von 1 bis 100 ist
oder durch Amidierung mit Diaminen der genannten allgemeinen Formel Ib, dadurch gekennzeichnet, daß je eine oder mehrere Verbindungen aus der Gruppe der Diamino- und Dicarbonsäure-Verbindungen polykondensiert werden.

2. Verfahren nach Anspruch 1, dadurch gekennzeichnet, daß man biologisch abbaubare Polyamide, wobei die Seitengruppe R¹ n- bzw. iso-(C₁-C₁₈)Alkyloxy, Methoxy(C₂-C₄)alkyloxy, Hydroxy(C₂-C₄)alkyloxy, (C₂-C₄)Alkoxycarbonylalkylenoxy, Trichlorisobutyloxy, Hydroxy (C₂-C₆)alkylamino, (C₂-C₄)Alkanoyloxyethylamino, Mercapto (C₂, C₄)alkylamino oder Methylester der natürlichen α-Aminosäuren bedeutet, herstellt.

3. Verfahren nach Anspruch 1, dadurch gekennzeichnet, daß man biologisch abbaubare Polyamide, wobei R¹ eine Methyl-, Ethyl- oder n-Butyloxygruppe bedeutet, herstellt.

4. Verfahren nach Anspruch 1, dadurch gekennzeichnet, daß man biologisch abbaubare Polyamide, wobei als Dicarbonsäuren geradkettige, gesättigte oder 1-fach ungesättigte Dicarbonsäuren mit 4-8 Kohlenstoffatomen eingesetzt werden, herstellt.

5. Verfahren nach Anspruch 1, dadurch gekennzeichnet, daß man biologisch abbaubare Polyamide, wobei als Dicarbonsäuren Fumarsäure und Glutarsäure eingesetzt werden, herstellt.

6. Verfahren nach Anspruch 1, dadurch gekennzeichnet, daß man biologisch abbaubare Polyamide, wobei die allgemeine Formel IIb 2,3-Butandiol bedeutet, herstellt.

7. Verwendung des Polyamids, hergestellt nach Anspruch 1 zum Einkapseln von biologisch aktiven Substanzen.

8. Verwendung des Polyamids, hergestellt nach Anspruch 1 zur Herstellung von biologisch abbaubaren Wirkstoffdepotzubereitungen mit kontrollierter Wirkstoffabgabe.

9. Verwendung des Polyamids, hergestellt nach Anspruch 1 in Abmischungen mit anderen bioabbaubaren Polyamiden oder physiologisch unbedenklichen Hilfsstoffen, zum Einkapseln von biologisch aktiven Substanzen.

10. Verwendung des Polyamids, hergestellt nach Anspruch 1 in Abmischungen mit anderen bioabbaubaren Polyamiden oder physiologisch unbedenklichen Hilfsstoffen, zur Herstellung von biologisch abbaubaren Wirkstoffdepotzubereitungen mit kontrollierter Wirkstoffabgabe.

## Claims (Claims for the following Contracting State(s): BE, CH, DE, FR, GB, GR, IT, LU, NL, SE)

1. A biologically degradable polyamide in which monomeric compounds derived from Ia and/or IIa are incorporated into the polymer backbone via two amino or carboxyl groups and carry in the α-position to the amide structure a functional group which is responsible for degradation and active compound release, and which essentially comprises 50 mol% of each of the repeating units I and II given below:
I) repeating units derived from diamino compounds from the group comprising
- the monomeric compounds of the formula Ia, 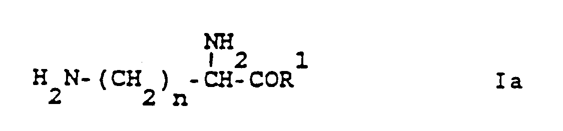 in which R¹ denotes a physiologically acceptable, hydrolyzable alkoxy group, having up to 18 carbon atoms, which can optionally be substituted by physiologically acceptable side groups, or denotes a physiologically acceptable, hydrolyzable alkylamino or aralkylamino group, and
n is 3 or 4,
and/or
- the monomeric compound which is produced by esterification of aminoethanol with the dicarboxylic acids of the citrate cycle, and/or
- the monomeric compound of the formula Ib, 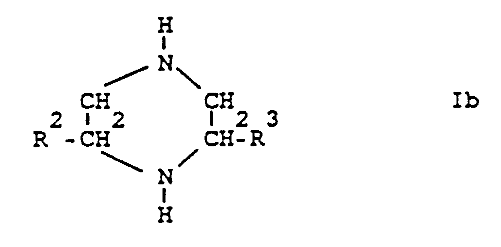
in which R² and R³, independently of one another, denote hydrogen or methyl, and
II) repeating units derived from a dicarboxylic acid compound from the group comprising
- the monomeric compound of the formula IIa, 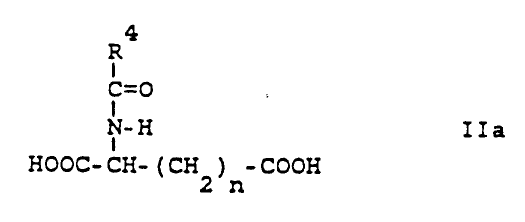 in which R⁴ denotes an alkyl group having 1 to 3 carbon atoms, and
n denotes 1 or 2, and/or
- the monomeric straight-chain, saturated or monounsaturated dicarboxylic acids having 2-10 carbon atoms, and/or
- the monomeric compound which is produced by esterification of the dicarboxylic acids of the citrate cycle with diols of the formula IIb, 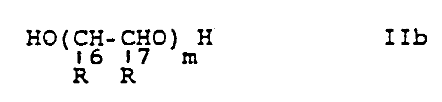 in which R⁶ and R⁷, independently of one another, denote hydrogen or a methyl group, and
m is a number in the range 1 to 100,
or by amidation with diamines of the formula Ib mentioned.

2. A polyamide as claimed in claim 1, wherein the side group R¹ denotes n- or iso-(C₁-C₁₈)alkoxy, methoxy(C₂-C₄)-alkoxy, hydroxy(C₂-C₄)alkoxy, (C₂-C₄)alkoxycarbonylalkyleneoxy, trichloroisobutoxy, hydroxy(C₂-C₆)alkylamino, (C₂-C₄)alkanoyloxyethylamino, mercapto(C₂-C₄)alkylamino or methyl esters of natural α-amino acids.

3. A polyamide as claim in claim 1, wherein R¹ denotes a methoxy, ethoxy or n-butoxy group.

4. A polyamide as claimed in claim 1, wherein the dicarboxylic acids employed are straight-chain, saturated or monounsaturated dicarboxylic acids having 4-8 carbon atoms.

5. A polyamide as claimed in claim 1, wherein the dicarboxylic acids employed are fumaric acid and glutaric acid.

6. A polyamide as claimed in claim 1, wherein the formula IIb denotes 2,3-butanediol.

7. A process for the preparation of a polyamide as claimed in claim 1, wherein one or more compounds from each of the groups comprising the diamino and dicarboxylic acid compounds are polycondensed.

8. The use of a polyamide as claimed in claim 1 for encapsulation of biologically active substances.

9. The use of a polyamide as claimed in claim 1 for the preparation of biologically degradable active compound depot preparations having controlled release of active compound.

10. The use of a polyamide as claimed in claim 1, in blends with other biodegradable polyamides or physiologically acceptable auxiliaries, for encapsulation of biologically active substances.

11. The use of a polyamide as claimed in claim 1, in blends with other biodegradable polyamides or physiologically acceptable auxiliaries, for the preparation of biologically degradable active compound depot preparations having controlled release of active compound.

## Claims (Claims for the following Contracting State(s): AT)

1. A process for the preparation of a biologically degradable polyamide in which monomeric compounds derived from Ia and/or IIa are incorporated into the polymer backbone via two amino or carboxyl groups and carry in the α-position to the amide structure a functional group which is responsible for degradation and active compound release, with
I) repeating units diamino compounds from the group comprising
- the monomeric compounds of the formula Ia, 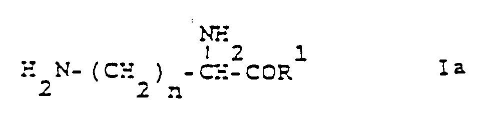 in which R¹ denotes a physiologically acceptable, hydrolyzable alkoxy group, having up to 18 carbon atoms, which can optionally be substituted by physiologically acceptable side groups, or denotes a physiologically acceptable, hydrolyzable alkylamino or aralkylamino group, and
n is 3 or 4,
and/or
- the monomeric compound which is produced by esterification of aminoethanol with the dicarboxylic acids of the citrate cycle, and/or
- the monomeric compound of the formula Ib, 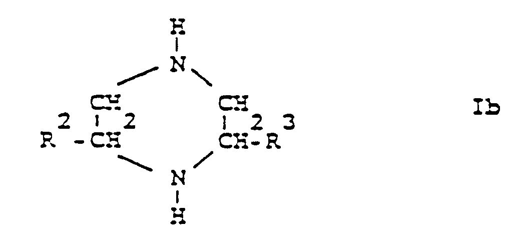
in which R² and R³, independently of one another, denote hydrogen or methyl, and
II) repeating units of dicarboxylic acid compound from the group comprising
- the monomeric compound of the formula IIa, 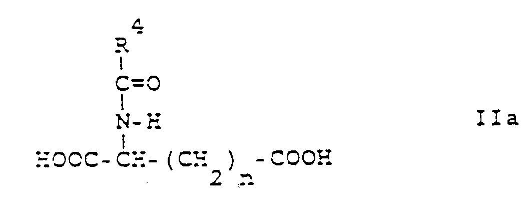 in which R⁴ denotes an alkyl group having 1 to 3 carbon atoms, and
n denotes 1 or 2, and/or
- the monomeric straight-chain, saturated or monounsaturated dicarboxylic acids having 2-10 carbon atoms, and/or
- the monomeric compound which is produced by esterification of the dicarboxylic acids of the citrate cycle with diols of the formula IIb, 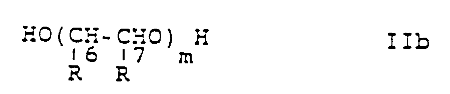 in which R⁶ and R⁷, independently of one another, denote hydrogen or a methyl group, and
m is a number in the range 1 to 100,
or by amidation with diamines of the formula Ib mentioned, wherein one or more compounds from each of the groups comprising the diamino and dicarboxylic acid compounds are polycondensed.

2. The process as claimed in claim 1, which comprises preparing a biologically degradable polyamide in which the side group R¹ denotes n- or iso-(C₁-C₁₈)-alkoxy, methoxy(C₂-C₄)-alkoxy, hydroxy(C₂-C₄)alkoxy, (C₂-C₄)-alkoxycarbonylalkyleneoxy, trichloroisobutoxy, hydroxy(C₂-C₆)alkylamino, (C₂-C₄)alkanoyloxyethylamino, mercapto(C₂-C₄)alkylamino or methyl esters of natural α-amino acids.

3. The process as claimed in claim 1, which comprises preparing a biologically degradable polyamide in which R¹ denotes a methoxy, ethoxy or n-butoxy group.

4. The process as claimed in claim 1, which comprises preparing a biologically degradable polyamide in which the dicarboxylic acids employed are straight-chain, saturated or monounsaturated dicarboxylic acids having 4-8 carbon atoms.

5. The process as claimed in claim 1, which comprises preparing a biologically degradable polyamide in which the dicarboxylic acids employed are fumaric acid and glutaric acid.

6. The process as claimed in claim 1, which comprises preparing a biologically degradable polyamide in which the formula IIb denotes 2,3-butanediol.

7. The use of a polyamide prepared as claimed in claim 1 for encapsulation of biologically active substances.

8. The use of a polyamide prepared as claimed in claim 1 for the preparation of biologically degradable active compound depot preparations having controlled release of active compound.

9. The use of a polyamide prepared as claimed in claim 1, in blends with other biodegradable polyamides or physiologically acceptable auxiliaries, for encapsulation of biologically active substances.

10. The use of a polyamide prepared as claimed in claim 1, in blends with other biodegradable polyamides or physiologically acceptable auxiliaries, for the preparation of biologically degradable active compound depot preparations having controlled release of active compound.

## Revendications (Revendications pour l'(les) Etat(s) contractant(s) suivant(s): BE, CH, DE, FR, GB, GR, IT, LI, LU, NL, SE)

1. Polyamide biologiquement dégradable, dans lequel des composés monomères dérivés de Ia et/ou IIa sont incorporés, par l'intermédiaire de deux groupes amino ou carboxy, dans le squelette du polymère et qui portent, en position α par rapport à la structure amide, un groupe fonctionnel responsable de la dégradation et de la libération de substance active, polyamide qui contient pratiquement 50 % de chacun des motifs répétitifs I et II,
les motifs répétitifs I) dérivés du composé diaminé choisi parmi
- les composés monomères de formule générale Ia 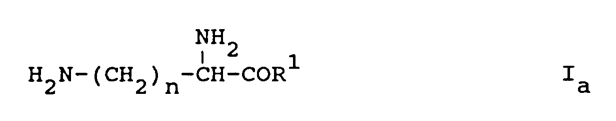 dans laquelle
R¹ représente un groupe alcoxy hydrolysable physiologiquement acceptable ayant jusqu'à 18 atomes de carbone, qui peut éventuellement être substitué par des groupes latéraux physiologiquement acceptables, ou
un groupe alkylamino ou aralkylamino hydrolysable, physiologiquement acceptable, et
n est 3 ou 4,
et/ou
- le composé monomère qui est formé par estérification de l'aminoéthanol avec les acides dicarboxyliques du cycle du citrate, et/ou
- le composé monomère de formule générale Ib 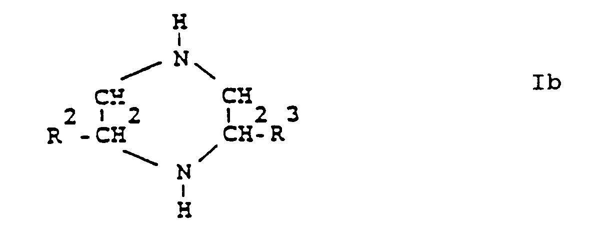 dans laquelle R² et R³ représentent, indépendamment l'un de l'autre, un atome d'hydrogène ou le groupe méthyle, et les motifs répétitifs II) dérivés du composé de type acide dicarboxylique de formule générale 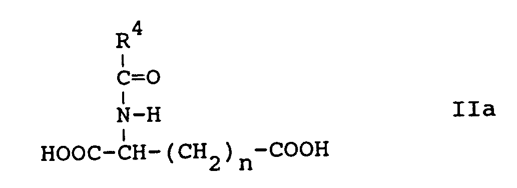 dans laquelle R⁴ représente un groupe alkyle ayant de 1 à 3 atomes de carbone et
n représente 1 ou 2 et/ou
- les acides dicarboxyliques monomères à chaîne droite, saturés ou à une insaturation ayant de 2 à 10 atomes de carbone, et/ou
- le composé monomère qui est formé par estérification des acides dicarboxyliques du cycle du citrate avec des diols de formule générale IIb, 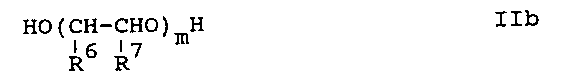 dans laquelle R⁶ et R⁷ représentent, indépendamment l'un de l'autre, un atome d'hydrogène ou le groupe méthyle, et
m représente un nombre allant de 1 à 100,
ou par amidation avec des diamines de formule générale Ib précitée.

2. Polyamide selon la revendication 1, caractérisé en ce que le groupe latéral R¹ représente un groupe n- ou isoalkyloxy en C₁-C₁₈, méthoxy-alkyloxy(C₂-C₄), hydroxyalkyloxy(C₂-C₄), alcoxy(C₂-C₄)-carbonylalkylène-oxy, trichlorisobutyloxy, hydroxy-alkyl(C₂-C₆)amino, alcanoyl(C₂-C₄)-oxyéthylamino, mercapto-alkyl(C₂-C₄)amino ou les esters méthyliques des α-aminoacides existant dans la nature.

3. Polyamide selon la revendication 1, caractérisé en ce que R¹ représente le groupe méthyle, éthyle ou n-butyloxy.

4. Polyamide selon la revendication 1, caractérisé en ce que, en tant qu'acides dicarboxyliques, on utilise des acides dicarboxyliques à chaîne droite, saturés ou à une insaturation, ayant de 4 à 8 atomes de carbone.

5. Polyamide selon la revendication 1, caractérisé en ce que, en tant qu'acides dicarboxyliques, on utilise l'acide fumarique et l'acide glutarique.

6. Polyamide selon la revendication 1, caractérisé en ce que la formule générale IIb représente le 2,3-butanediol.

7. Procédé pour la préparation du polyamide selon la revendication 1, caractérisé en ce que l'on soumet à une polycondensation un ou plusieurs composés appartenant au groupe des composés diaminés et dicarboxylés.

8. Utilisation du polyamide selon la revendication 1, pour l'encapsulation de substances biologiquement actives.

9. Utilisation du polyamide selon la revendication 1, pour la préparation de compositions retard de substances actives, biologiquement dégradables, à libération programmée de la substance active.

10. Utilisation du polyamide selon la revendication 1, dans des mélanges avec d'autres polyamides biodégradables ou adjuvants physiologiquement acceptables, pour l'encapsulation de substances biologiquement actives.

11. Utilisation du polyamide selon la revendication 1, dans des mélanges avec d'autres polyamides biodégradables ou adjuvants physiologiquement acceptables, pour la préparation de compositions retard de substances actives, biologiquement dégradables, à libération programmée de la substance active.

## Revendications (Revendications pour l'(les) Etat(s) contractant(s) suivant(s): AT)

1. Procédé pour la préparation d'un polyamide biologiquement dégradable, dans lequel des composés monomères dérivés de Ia et/ou IIa sont incorporés, par l'intermédiaire de deux groupes amino ou carboxy, dans le squelette du polymère et qui portent, en position α par rapport à la structure amide, un groupe fonctionnel responsable de la dégradation et de la libération de substance active, polyamide qui contient pratiquement 50 % de chacun des motifs répétitifs I et II,
les motifs répétitifs I) dérivés du composé diaminé choisi parmi
- les composés monomères de formule générale Ia 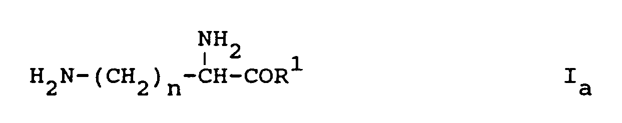 dans laquelle
R¹ représente un groupe alcoxy hydrolysable physiologiquement acceptable ayant jusqu'à 18 atomes de carbone, qui peut éventuellement être substitué par des groupes latéraux physiologiquement acceptables, ou
un groupe alkylamino ou aralkylamino hydrolysable, physiologiquement acceptable, et
n est 3 ou 4,
et/ou
- le composé monomère qui est formé par estérification de l'aminoéthanol avec les acides dicarboxyliques du cycle du citrate, et/ou
- le composé monomère de formule générale Ib 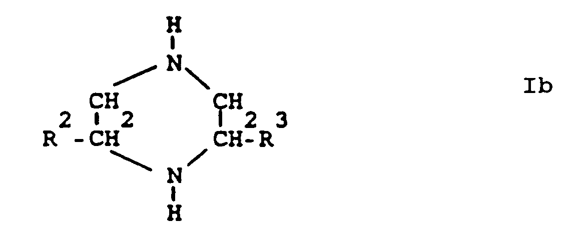 dans laquelle R² et R³ représentent, indépendamment l'un de l'autre, un atome d'hydrogène ou le groupe méthyle, et les motifs répétitifs II) dérivés de composés de type acide dicarboxylique de formule générale 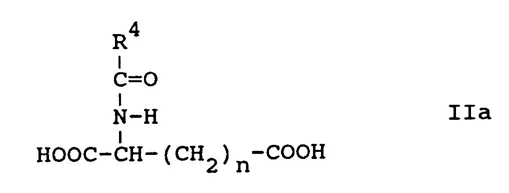 dans laquelle R⁴ représente un groupe alkyle ayant de 1 à 3 atomes de carbone et
n représente 1 ou 2 et/ou
- les acides dicarboxyliques monomères à chaîne droite, saturés ou à une insaturation ayant de 2 à 10 atomes de carbone, et/ou
- le composé monomère qui est formé par estérification des acides dicarboxyliques du cycle du citrate avec des diols de formule générale IIb, 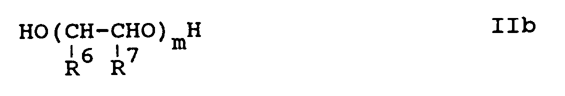 dans laquelle R⁶ et R⁷ représentent, indépendamment l'un de l'autre, un atome d'hydrogène ou le groupe méthyle, et
m représente un nombre allant de 1 à 100,
ou par amidation avec des diamines de formule générale Ib précitée,
caractérisé en ce que l'on soumet à une polycondensation un ou plusieurs composés appartenant au groupe des composés diaminés et dicarboxylés.

2. Procédé selon la revendication 1, caractérisé en ce que l'on prépare des polyamides biologiquement dégradables dans lesquels le groupe latéral R¹ représente un groupe n- ou isoalkyloxy en C₁-C₁₈, méthoxy-alkyloxy(C₂-C₄), hydroxy-alkyloxy(C₂-C₄), alcoxy(C₂-C₄)-carbonylalkylène-oxy, trichlorisobutyloxy, hydroxy-alkyl(C₂-C₆)amino, alcanoyl(C₂-C₄)-oxyéthylamino, mercapto-alkyl(C₂-C₄)amino ou les esters méthyliques des α-aminoacides existant dans la nature.

3. Procédé selon la revendication 1, caractérisé en ce que l'on prépare des polyamides biologiquement dégradables dans lesquels R¹ représente le groupe méthyle, éthyle ou n-butyloxy.

4. Procédé selon la revendication 1, caractérisé en ce que l'on prépare des polyamides biologiquement dégradables dans lesquels, en tant qu'acides dicarboxyliques, on utilise des acides dicarboxyliques à chaîne droite, saturés ou à une insaturation, ayant de 4 à 8 atomes de carbone.

5. Procédé selon la revendication 1, caractérisé en ce que l'on prépare des polyamides biologiquement dégradables dans lesquels, en tant qu'acides dicarboxyliques, on utilise l'acide fumarique et l'acide glutarique.

6. Procédé selon la revendication 1, caractérisé en ce que l'on prépare des polyamides biologiquement dégradables dans lesquels la formule générale IIb représente le 2,3-butanediol.

7. Utilisation du polyamide préparé selon la revendication 1, pour l'encapsulation de substances biologiquement actives.

8. Utilisation du polyamide préparé selon la revendication 1, pour la préparation de compositions retard de substances actives, biologiquement dégradables, à libération programmée de la substance active.

9. Utilisation du polyamide préparé selon la revendication 1, dans des mélanges avec d'autres polyamides biodégradables ou adjuvants physiologiquement acceptables, pour l'encapsulation de substances biologiquement actives.

10. Utilisation du polyamide préparé selon la revendication 1, dans des mélanges avec d'autres polyamides biodégradables ou adjuvants physiologiquement acceptables, pour la préparation de compositions retard de substances actives, biologiquement dégradables, à libération programmée de la substance active.
